# FASCICULE DE BREVET EUROPEEN

(11) **EP 1 035 875 B1**
(45) Date de publication et mention de la délivrance du brevet: **14.05.2003**
(21) Numéro de dépôt: 98956978.5
(22) Date de dépôt: 27.11.1998
(51) Int. Cl.: A61L 2/04

(54) **PROCEDE DE DECONTAMINATION D'UNE AIGUILLE CREUSE ET AIGUILLE CREUSE**
VERFAHREN ZUM DEKONTAMINIEREN VON HOHLNADELN UND HOHLNADEL HIERZU
METHOD FOR DECONTAMINATING A HOLLOW NEEDLE AND HOLLOW NEEDLE THEREFOR

(30) Priorité: 27.11.1997 FR 9715185
(43) Date de publication de la demande: 20.09.2000
(73) Titulaire: BIO MERIEUX, 69280 Marcy l'Etoile (FR)
(72) Inventeur: COLIN, Bruno, F-69280 Marcy l'Etoile (FR); PRIVAT, Marie, F-69560 Saint Romain en Gal (FR)
(74) Mandataire: Guerre, Dominique
(86) Numéro de dépôt international: FR9802561
(87) Numéro de publication internationale: WO99027973

(56) Documents cités:
- EP-A- 0 727 228
- FR-A- 2 091 884
- FR-A- 2 618 336
- US-A- 3 742 187

## Description

La présente invention concerne un procédé de décontamination d'une aiguille creuse métallique destinée au prélèvement et/ou à la distribution d'un liquide contaminateur, l'aiguille coopérant avec des moyens de branchement électrique permettant d'établir un courant électrique dans l'aiguille pour décontaminer et préserver l'intégrité de l'aiguille en vue de sa réutilisation.

La présente invention concerne également une aiguille creuse comprenant un dispositif de décontamination qui permet la mise en oeuvre du procédé ci-dessus.

Par liquide contaminateur, on entend tout milieu appelé à être traité ou manipulé dans tout procédé, processus ou méthode par exemple d'analyse, mais devant être substantiellement éliminé ou supprimé, car indésirable à un moment ou à un autre dudit processus, procédé ou méthode, et ceci quelle que soit la forme physique ou présentation dudit milieu, par exemple qu'il s'agisse d'un matériau, matière ou échantillon sous forme liquide ou gazeuse ou diphasique (liquide + gaz) ou encore sous forme de poudre, dès lors que ledit milieu peut être manipulé comme un fluide notamment par aspiration et/ou refoulement.

En conséquence de la définition précédente, les termes « contaminé » et « décontaminé » signifient respectivement « mettre au contact ou apporter un liquide contaminateur dans ou sur un objet » et « éliminer ou supprimer le même liquide contaminateur dudit objet ».

Au premier rang des liquides contaminateurs considérés par la présente invention, figurent les milieux biologiques, tels qu'échantillons de fluide ou prélèvements corporels susceptibles de contenir ou être souillés par différents germes non pathogènes tels que virus, bactéries ou d'autres cellules. Dans ce cas et pour la description ci-après, « liquide contaminateur » signifiera « liquide non stérile » et « décontaminé » signifiera « stérilisé ».

Mais la présente invention ne saurait être limitée à la stérilisation puisque bien d'autres milieux contaminants peuvent être considérés selon l'invention. Ainsi, tout matériel ou substance nucléique telle que l'ADN manipulée dans toute technique de biologie moléculaire peut faire l'objet de la présente invention. C'est également le cas de tout milieu organique qu'il est nécessaire à la fois de manipuler et d'éliminer- à un moment ou à un autre de tout procédé, par exemple d'analyse chimique ou biochimique.

Cependant, la caractéristique commune de tous les liquides contaminateurs considérés par la présente invention est que ceux-ci peuvent être détruits en portant la température à une valeur relativement élevée, par exemple excédant 150°C.

L'état de la technique a déjà proposé de procédés ou dispositifs pour décontaminer efficacement les aiguilles creuses métalliques. Néanmoins, la plupart des documents de l'état de la technique, tels que GB-A-2.211.420, US-A-5.375.200 ou EP-A-0.136.392, ont pour objet de détruire les aiguilles qui ont été utilisées.

Une telle technique n'est absolument pas utilisable avec un automate car elle augmente considérablement le coût d'utilisation de tels automates, du fait de la destruction de très nombreuses aiguilles.

Une autre solution particulièrement évidente consiste donc à changer les embouts qui sont utilisés pour les pipettes, technique qui peut être adaptée pour les automates.

Dans ce cas, il va y avoir un nombre considérable de manipulations, ce qui va entraîner une perte de temps, d'une part, et une perte d'argent, d'autre part.

La dernière technique consiste à effectuer un lavage. La plupart des techniques actuelles nécessitent une grande quantité de liquide pour permettre le nettoyage d'une seule aiguille creuse. En moyenne il faut compter environ 10 ml de liquide de nettoyage pour permettre une réelle décontamination.

Néanmoins, on a pu obtenir des résultats sensiblement meilleurs par d'autres procédés, tel que celui exposé dans la demande de brevet EP-A-0.727.228 du présent demandeur. Celui-ci concerne un procédé de chauffage d'une aiguille creuse consistant essentiellement en un tube métallique, dont l'intérieur et/ou l'extérieur ont été mis préalablement au contact d'un milieu contaminant, selon lequel on apporte un courant électrique dans ladite aiguille circulant dans la paroi métallique et selon la longueur dudit tube, et dissipant dans ce dernier une énergie thermique par effet ohmique. Ce procédé est caractérisé en ce qu'on apporte dans ladite aiguille une quantité prédéterminée d'énergie électrique dosée en fonction des caractéristiques électriques dudit tube, d'une part pour suffire à la décontamination de l'aiguille tant intérieure qu'extérieure, et d'autre part pour limiter le chauffage et préserver l'intégrité, dont la forme originelle de ladite aiguille.

La quantité totale de liquide de nettoyage est, dans ce cas, largement inférieure à toutes les techniques précédemment décrites. Néanmoins, l'application d'une charge électrique, par exemple au niveau de l'aiguille où le liquide contaminateur est présent, peut entraîner la cristallisation de certains éléments de ce liquide contaminateur, nuisant ainsi à la qualité de la décontamination effectuée.

La présente invention a donc pour objet de proposer un procédé de décontamination d'une aiguille creuse mais également une aiguille creuse comprenant un dispositif de décontamination d'une telle aiguille creuse qui nécessite peu de liquide de nettoyage, le procédé et l'aiguille creuse éliminent les solides contaminés (embouts jetables) et réduisent les volumes d'effluents (liquides de nettoyage contaminés).

A cet effet, la présente invention concerne un procédé de décontamination d'une aiguille creuse métallique destinée au prélèvement et/ou à la distribution d'un liquide contaminateur, l'aiguille coopérant avec des moyens de branchement électrique permettant d'établir un courant électrique dans l'aiguille pour décontaminer et préserver l'intégrité de l'aiguille en vue de sa réutilisation, consistant :
- à injecter un liquide de nettoyage dans l'aiguille creuse ayant effectuée le prélèvement et/ou la distribution du liquide contaminateur,
- à appliquer une source de chaleur au niveau de tout ou partie du liquide de nettoyage présent dans ladite aiguille creuse entre les moyens de branchement, et
- à éjecter ledit liquide contaminateur présent ainsi que tout ou partie dudit liquide de nettoyage.

Selon un premier mode de réalisation, l'injection du liquide de nettoyage et l'application du courant électrique se font simultanément.

Selon un deuxième mode de réalisation, l'injection du liquide de nettoyage s'effectue préalablement à l'application de la source de chaleur.

Selon un troisième mode de réalisation, l'application de la source de chaleur s'effectue préalablement à l'injection du liquide de nettoyage.

Le courant électrique vaporise une partie du liquide de nettoyage présent dans ladite aiguille creuse.

Selon un mode particulièrement intéressant de réalisation, la source de chaleur est due à un courant électrique.

Selon un mode particulièrement intéressant de réalisation du procédé, l'éjection s'effectue sous l'action de la pression dudit liquide de nettoyage vaporisé.

La présente invention concerne également une aiguille creuse métallique comprenant un dispositif de décontamination et destinée au prélèvement et/ou à la distribution d'un liquide contaminateur, l'aiguille coopérant avec des moyens de branchement électrique permettant d'établir un courant électrique dans l'aiguille pour décontaminer et préserver l'intégrité de l'aiguille en vue de sa réutilisation, le dispositif de décontamination comportant au moins deux fils électriques, chaque fil reliant une source d'énergie électrique à l'aiguille ; les points d'implantation desdits fils, formant électrodes, sont différents l'un de l'autre le long de ladite aiguille, mais également de l'extrémité libre de l'aiguille, et que l'autre extrémité de ladite aiguille coopère avec un moyen de distribution d'un liquide de nettoyage.

Le liquide contaminateur, après le prélèvement et/ou la distribution, est présent entre l'extrémité libre de l'aiguille creuse et l'électrode la plus proche de ladite extrémité libre.

Le liquide de nettoyage, après le prélèvement et/ou la distribution, est présent entre les deux électrodes.

Selon un mode particulier de réalisation, une bulle d'air est présente entre le liquide contaminateur et le liquide de nettoyage.

Selon un autre mode de réalisation, le liquide de nettoyage est constitué par de l'eau.

Selon un encore un autre mode de réalisation de la présente invention, le liquide de nettoyage est constitué par une solution tampon.

La présente invention sera décrite en rapport avec les figures ci-jointes qui n'ont pas de caractère limitatif mais représentent un mode particulier de réalisation de ladite invention.

Le dispositif de décontamination de l'aiguille selon l'invention est associé à un dispositif de nettoyage externe de l'aiguille creuse.

La figure 1 représente une vue schématique d'une aiguille creuse comprenant un dispositif de décontamination selon la présente invention.

La figure 2 représente une vue identique à la figure 1, lorsque le liquide contaminateur vient d'être prélevé.

La figure 3 représente une vue identique aux figures précédentes, dans laquelle le liquide de nettoyage est vaporisé sous l'action d'une décharge de courant électrique associée à l'injection de liquide de nettoyage supplémentaire.

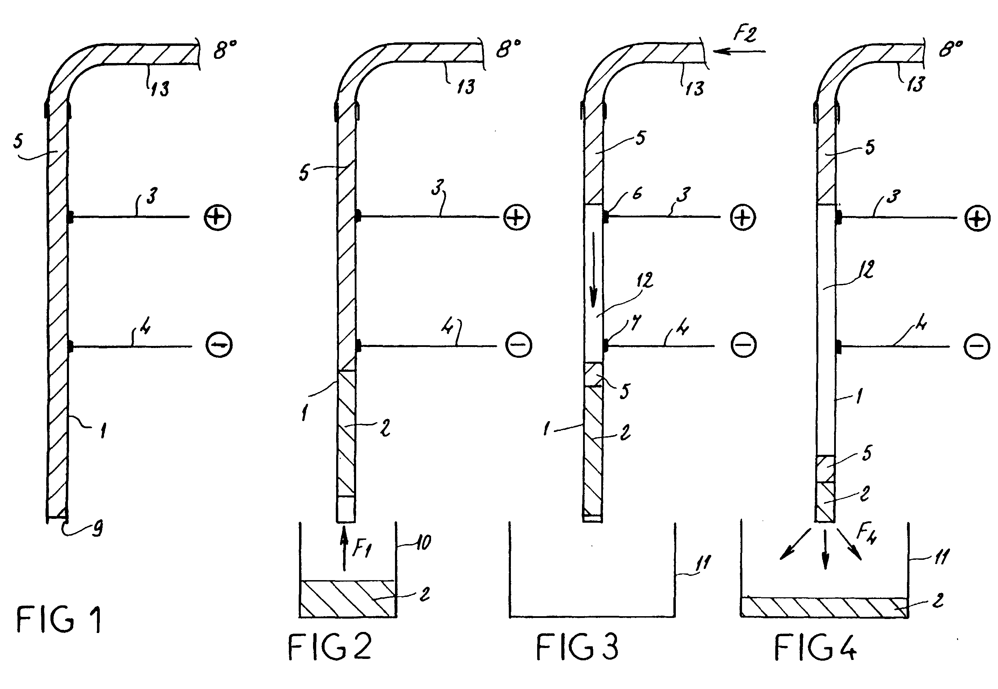

Enfin, la figure 4 représente une vue identique aux figures précédentes, lors de l'éjection du liquide contaminateur et d'une partie du liquide de nettoyage.

La présente invention concerne un procédé de décontamination d'une aiguille creuse 1 bien représenté sur l'ensemble des figures 1 à 4. Cette aiguille creuse 1 est en position sensiblement verticale et comporte une extrémité libre 9 en position inférieure, alors que son extrémité supérieure coopère avec un moyen de distribution 8 d'un liquide de nettoyage 5.

Sur les figures, seule une conduite 13 souple du moyen de distribution 8 du liquide de nettoyage 5 est représentée, le reste du moyen de distribution étant symboliquement représenté par la référence 8 sur les figures.

Quoi qu'il en soit, ce moyen de distribution 8 est essentiellement constitué d'un réservoir, contenant le liquide de nettoyage 5, qui est associé d'une part à un élément de distribution, tel qu'une seringue, et à une électrovanne permettant ou interdisant le fonctionnement de l'élément de distribution.

L'aiguille creuse 1 est en matière métallique et comporte sur sa paroi deux électrodes 6 et 7 qui sont reliées, par l'intermédiaire de branchements électriques 3 et 4, à une source électrique.

Dans sa position de repos, l'aiguille est telle que représentée à la figure 1, c'est-à-dire que l'aiguille creuse 1 ne contient que du liquide de nettoyage 5, ce liquide de nettoyage 5 pouvant être constitué par de l'eau ou une solution tampon.

Selon la figure 2, il sera possible par l'intermédiaire de l'élément de distribution d'aspirer, selon F1, une certaine quantité de liquide contaminant 2 contenu dans un récipient 10.

Lorsque le liquide contaminant 2 est contenu dans l'aiguille creuse 1, il y aura la possibilité de le relâcher pour permettre une réaction ou une manipulation. De ce fait, l'aiguille 1 sera souillée et il y aura nécessité de la décontaminer.

Sur les figures et afin de mieux visualiser la décontamination effectuée, le liquide contaminant 2 n'est pas relâché, et c'est l'ensemble du liquide contaminant 2 qui va faire l'objet de la décontamination.

Pour ce faire, on applique une différence de potentiel sur une partie de l'aiguille creuse 1, c'est-à-dire entre les deux électrodes 6 et 7. A ce niveau, le liquide de nettoyage 5 est vaporisé 12. Simultanément, on envoie, par injection selon F2, du liquide de nettoyage 9 dans l'aiguille creuse 1, de sorte que le gaz 12 ne peut être évacué, du fait de l'augmentation de volume entre la quantité de liquide 5, qui va être vaporisé, et la quantité de liquide vaporisé 12, que par l'extrémité libre 9, sous l'action F3 du gaz. De ce fait, comme cela est représenté à la figure 4, le liquide contaminateur 2 est éjecté selon F4 dans un récipient 11, prévu à cet effet. Une partie du liquide de nettoyage peut éventuellement être associée à ce liquide contaminateur résiduel.

Pour que cela soit réalisable, l'électrovanne, qui permet le passage selon F2 du liquide de nettoyage 5, est, juste après cette injection, fermée. De sorte que le liquide de nettoyage vaporisé 12 dirige obligatoirement le liquide contaminateur 2, situé en aval en direction de l'extrémité libre 9 de l'aiguille creuse 1.

Lors de ce procédé, il y a une nécessité que le liquide contaminant 2 ne se trouve pas présent entre les deux électrodes 6 et 7 ; de ce fait, il est nécessaire que l'électrode située en position inférieure, qui est l'électrode négative 7, soit suffisamment éloignée de l'extrémité libre 9 de l'aiguille 1, pour que le liquide contaminant 2 ne vienne jamais à son niveau. Ceci étant réalisé, le liquide de nettoyage 5, qui sera vaporisé 12, permet l'éjection tout à fait naturellement du liquide contaminant 2 et assure ainsi une décontamination de l'aiguille creuse 1.

Cette décontamination étant très efficace, elle le sera d'autant plus pour de plus faible quantité de liquide contaminant 2.

Bien entendu, il est possible d'associer ce nettoyage interne de l'aiguille creuse 1 à un moyen de nettoyage externe déjà bien connu de l'état de la technique, tel qu'un dispositif de nettoyage par les effluents ou par un liquide annexe.

Le fait que l'électrode négative 7 soit positionnée au plus près de l'extrémité libre 9 de l'aiguille 1 n'est pas obligatoire, ce pourrait être l'électrode positive 6. De même, il est possible que le courant électrique fourni soit du courant alternatif ou du courant continu.

Il est également possible que ledit courant électrique soit remplacé par n'importe quelle source de chaleur, comme par exemple un laser, une flamme, une torche à plasma.

Enfin, la simultanéité entre l'application de la source de chaleur et l'injection du liquide de nettoyage 5 doit être interprétée comme correspondant à un écart de temps de quelques dixièmes de seconde à quelques centièmes de seconde, voire quelques millièmes de seconde Néanmoins, il est également possible que l'application de la source de chaleur précède l'injection du liquide 5 et vice versa.

### REFERENCES

1. Aiguille creuse
2. Liquide contaminateur
3. Moyen de branchement électrique
4. Moyen de branchement électrique
5. Liquide de nettoyage
6. Electrode positive
7. Electrode négative
8. Moyen de distribution du liquide de nettoyage 5
9. Extrémité libre de l'aiguille 1
10. Récipient contenant le liquide contaminateur 2
11. Récipient contenant le ou les liquide(s) contaminateur 2 et/ou de nettoyage 5 résiduel(s)
12. Liquide de nettoyage vaporisé
13. Conduite du liquide de nettoyage 5
F1. Aspiration du liquide contaminateur 2 par l'aiguille 1
F2. Injection de liquide de nettoyage 5 dans l'aiguille 1
F3. Action du liquide de nettoyage vaporisé
F4. Ejection du ou des liquide(s) contaminateur 2 et/ou de nettoyage 5 résiduel(s)

## Revendications

1. Procédé de décontamination d'une aiguille creuse métallique (1) destinée au prélèvement et/ou à la distribution d'un liquide contaminateur (2), l'aiguille (1) coopérant avec des moyens de branchement électrique (3 et 4) permettant d'établir un courant électrique dans l'aiguille (1) pour décontaminer et préserver l'intégrité de ladite aiguille (1) en vue de sa réutilisation, consistant :
- à injecter un liquide de nettoyage (5) dans l'aiguille creuse (1), ayant effectué le prélèvement et/ou la distribution du liquide contaminateur (2),
- à appliquer une source de chaleur au niveau de tout ou partie du liquide de nettoyage (5) présent dans ladite aiguille creuse (1) entre les moyens de branchement (3 et 4), et
- à éjecter ledit liquide contaminateur (2) présent ainsi que tout ou partie dudit liquide de nettoyage (5).

2. Procédé, selon la revendication 1, **caractérisé en ce que** l'injection du liquide de nettoyage (5) et l'application de la source de chaleur se font simultanément.

3. Procédé, selon la revendication 1, **caractérisé en ce que** l'injection du liquide de nettoyage (5) s'effectue préalablement à l'application de la source de chaleur.

4. Procédé, selon la revendication 1, **caractérisé en ce que** l'application de la source de chaleur s'effectue préalablement à l'injection du liquide de nettoyage (5).

5. Procédé, selon l'une quelconque des revendications 1 à 4, **caractérisé en ce que** l'effet Joule généré par le courant électrique vaporise tout ou partie du liquide de nettoyage (5) présent dans ladite aiguille creuse (1).

6. Procédé, selon l'une quelconque des revendications 1 à 5, **caractérisé en ce que** l'éjection s'effectue sous l'action de la pression dudit liquide de nettoyage (5) vaporisé (12).

7. Aiguille creuse métallique (1) comprenant un dispositif de décontamination et destinée au prélèvement et/ou à la distribution d'un liquide contaminateur (2), l'aiguille (1) coopérant avec des moyens de branchement électrique (3 et 4) permettant d'établir un courant électrique dans l'aiguille (1) pour décontaminer et préserver l'intégrité de ladite aiguille (1) en vue de sa réutilisation, le dispositif de décontamination comportant au moins deux fils électriques (3 et 4), chaque fil (3 ou 4) reliant une source d'énergie électrique à l'aiguille (1) ; les points d'implantation desdits fils (3 et 4), formant électrodes (6 et 7), sont différents l'un de l'autre le long de ladite aiguille (1), mais également de l'extrémité libre (9) de l'aiguille (1), et que l'autre extrémité de ladite aiguille (1) coopère avec un moyen de distribution (8) d'un liquide de nettoyage (5)

8. Utilisation de l'aiguille selon la revendication 7, **caractérisée par le fait que** le liquide contaminateur (2), après le prélèvement et/ou la distribution, est présent entre l'extrémité libre (9) de l'aiguille creuse (1) et l'électrode (7) la plus proche de ladite extrémité libre (9).

9. Utilisation de l'aiguille selon la revenication 7, ou utilisation selon la revendication 8, **caractérisée par le fait que** le liquide de nettoyage (5), après le prélèvement et/ou la distribution, est présent entre les deux électrodes (6 et 7).

10. Utilisation de l'aiguille selon la revendication 7, ou utilisation selon l'une quelconque des revendications 8 et 9, **caractérisée par le fait qu'**une bulle d'air est présente entre le liquide contaminateur (2) et le liquide de nettoyage (5).

11. Utilisation de l'aiguille selon la revendication 7, ou utilisation selon l'une quelconque des revendications 8 à 10, **caractérisée par le fait que** le liquide de nettoyage (5) est constitué par de l'eau

12. Utilisation de l'aiguille selon la revendication 7, ou utilisation selon l'une quelconque des revendications 8 à 11, **caractérisée par le fait que** le liquide de nettoyage (5) est constitué par une solution tampon.

13. Ensemble de décontamination comprenant une aiguille selon la revendication 7, **caractérisé par le fait que** ledit dispositif est associé à un dispositif de nettoyage externe de l'aiguille creuse (1).

## Patentansprüche

1. Verfahren zur Dekontamination einer metallischen Hohlnadel (1), die für die Entnahme und Abgabe einer kontaminierenden Flüssigkeit (2) vorgesehen ist, wobei die Nadel (1) mit Mitteln zum Stromanschluss (3 und 4) zusammen wirkt, welche es ermöglichen, einen elektrischen Strom in der Nadel (1) zu verursachen, um diese Nadel (1) zu dekontaminieren und ihre Integrität im Hinblick auf deren Wiederverwendung aufrecht zu erhalten, bestehend aus:
- dem Einbringen einer Reinigungsflüssigkeit (5) in die Hohlnadel (1), durch welche die Entnahme und/oder Abgabe einer kontaminierenden Flüssigkeit (2) erfolgt ist,
- der Anwendung einer Wärmequelle im Bereich eines Teils oder der gesamten Reinigungsflüssigkeit (5), welche sich zwischen den Anschlussmitteln (3 und 4) in dieser Hohlnadel (1) befindet, und
- dem Ausstoß der vorhandenen kontaminierenden Flüssigkeit (2) sowie eines Teils oder der gesamten Reinigungsflüssigkeit (5).

2. Verfahren nach Anspruch 1, **dadurch gekennzeichnet, dass** das Einbringen der Reinigungsflüssigkeit (5) und die Anwendung der Wärmequelle gleichzeitig geschehen.

3. Verfahren nach Anspruch 1, **dadurch gekennzeichnet, dass** das Einbringen der Reinigungsflüssigkeit (5) vor der Anwendung der Wärmequelle erfolgt.

4. Verfahren nach Anspruch 1, **dadurch gekennzeichnet, dass** die Anwendung der Wärmequelle vor dem Einbringen der Reinigungsflüssigkeit (5) erfolgt.

5. Verfahren nach einem der Ansprüche 1 bis 4, **dadurch gekennzeichnet, dass** die Joulesche Wärme, die durch den elektrischen Strom erzeugt wurde, die in der Hohlnadel (1) befindliche Reinigungsflüssigkeit (5) zum Teil oder ganz verdampft.

6. Verfahren nach einem der Ansprüche 1 bis 5, **dadurch gekennzeichnet, dass** der Ausstoß unter dem Einfluss des Drucks der verdampften (12) Reinigungsflüssigkeit (5) erfolgt.

7. Metallische Hohlnadel (1), welche eine Vorrichtung zur Dekontamination aufweist und welche zur Entnahme und/oder Abgabe einer kontaminierenden Flüssigkeit (2) vorgesehen ist, wobei die Nadel (1) mit Mitteln zum Stronanschluss (3 und 4) zusammen wirkt, welche es ermöglichen, einen elektrischen Strom in der Nadel (1) herzustellen, um sie zu dekontaminieren und um die Integrität der Nadel (1), im Hinblick auf deren Wiederverwendung zu erhalten, und wobei die Vorrichtung zur Dekontamination aus zumindest zwei elektrischen Drähten (3 und 4) besteht, wobei jeder Draht (3 oder 4) eine elektrische Energiequelle mit der Nadel (1) verbindet; die Anbringungspunkte der Drähte (3 und 4), welche die Elektroden (6 und 7) bilden, sind unterschiedlich voneinander längs der Länge der Nadel (1), aber auch von offenen Ende (9) der Nadel (1) und wobei das andere Ende dieser Nadel (1) mit einem Mittel zur Abgabe (8) einer Reinigungsflüssigkeit (5) zusammen wirkt.

8. Verwendung der Nadel nach Anspruch 7, **dadurch gekennzeichnet, dass** die kontaminierende Flüssigkeit (2) nach der Entnahme und/oder Abgabe zwischen dem offenen Ende (9) der Hohlnadel (1) und der diesem offenen Ende (9) nächetgelegenen Elektrode (7) vorhanden ist.

9. Verwendung der Nadel nach Anspruch 7 oder Verwendung nach Anspruch 8, **dadurch gekennzeichnet, dass** die Reinigungsflüssigkeit (5) nach der Entnahme und/oder Abgabe zwischen den beiden Elektroden (6 und 7) vorhanden ist.

10. Verwendung der Nadel nach Anspruch 7 oder Verwendung nach einem der Ansprüche 8 und 9, **dadurch gekennzeichnet, dass** eine Luftblase zwischen der kontaminierenden Flüssigkeit (2) und der Reinigungsflüssigkeit (5) vorhanden ist.

11. Verwendung der Nadel nach Anspruch 7 oder Verwendung nach einem der Ansprüche 8 bis 10, **dadurch gekennzeichnet, dass** die Reinigungsflüssigkeit (5) Wasser ist.

12. Verwendung der Nadel nach Anspruch 7 oder Verwendung nach einem der Ansprüche 8 bis 11, **dadurch gekennzeichnet, dass** die Reinigungsflüssigkeit (5) eine Pufferlösung ist.

13. Zusammenbau zur Dekontamination, welcher eine Nadel nach Anspruch 7 aufweist, **dadurch gekennzeichnet, dass** diese Vorrichtung mit einer Vorrichtung zur äußeren Reinigung der Hohlnadel (1) verbunden ist.

## Claims

1. Method for decontaminating a hollow metal needle (1) intended for sampling and/or dispensing a contaminating liquid (2), the needle (1) cooperating with electrical wiring means (3 and 4) for providing an electric current in the needle (1) for decontaminating and preserving the integrity of the said needle (1) with a view to its re-use, consisting:
- in injecting a cleaning fluid (5) into the hollow needle (1), having carried out the sampling and/or dispensing of the contaminating liquid (2),
- in applying a heat source to all or part of the cleaning fluid (5) present in the said hollow needle (1) between the wiring means (3 and 4), and
- in ejecting the said contaminating liquid (2) present and all or part of the said cleaning fluid (5).

2. Method according to Claim 1, **characterized in that** the injection of the cleaning fluid (5) and the application of the heat source are done simultaneously.

3. Method according to Claim 1, **characterized in that** the injection of the cleaning fluid (5) takes place prior to the application of the heat source.

4. Method according to Claim 1, **characterized in that** the application of the heat source takes place prior to the injection of the cleaning fluid (5).

5. Method according to any one of Claims 1 to 4, **characterized in that** the Joule effect generated by the electric current vaporizes all or part of the cleaning fluid (5) present in the said hollow needle (1).

6. Method according to any one of Claims 1 to 5, **characterized in that** the ejection is carried out under the action of the pressure of the said vaporized (12) cleaning fluid (5).

7. Hollow metal needle (1) comprising a decontamination device and intended for sampling and/or dispensing a contaminating liquid (2), the needle (1) cooperating with electrical wiring (3 and 4) means for providing an electric current in the needle (1) for decontaminating and preserving the integrity of the said needle (1) with a view to its re-use, the decontamination device comprising at least two electrical wires (3 and 4), each wire (3 or 4) connecting a source of electrical energy to the needle (1); the implantation points of the said wires (3 and 4), which form electrodes (6 and 7), being different from each other along the length of the said needle (1), but also from the free end (9) of the needle (1), and the other end of the said needle (1) cooperating with a means (8) for dispensing a cleaning fluid (5).

8. Use of the needle according to Claim 7, **characterized in that** the contaminating liquid (2), after the sampling and/or the dispensing, is present between the free end (9) of the hollow needle (1) and the electrode (7) which is closest to the said free end (9).

9. Use of the needle according to Claim 7, or use according to claim 8, **characterized in that** the cleaning fluid (5), after the sampling and/or the dispensing, is present between the two electrodes (6 and 7).

10. Use of the needle according to Claim 7, or use according to either of Claims 8 and 9, **characterized in that** an air bubble is present between the contaminating liquid (2) and the cleaning fluid (5).

11. Use of the needle according to Claim 7, or use according to any one of Claims 8 to 10, **characterized in that** the cleaning fluid (5) consists of water.

12. Use of the needle according to Claim 7, or use according to any one of Claims 8 to 11, **characterized in that** the cleaning fluid (5) consists of a buffer solution.

13. Decontamination set comprising a needle according to Claim 7, **characterized in that** the said device is associated with a device for external cleaning of the hollow needle (1).
